# EUROPEAN PATENT SPECIFICATION

(11) **EP 1 810 033 B1**
(45) Date of publication and mention of the grant of the patent: **21.01.2009**
(21) Application number: 05804673.1
(22) Date of filing: 29.09.2005
(51) Int. Cl.: G01N 33/569

(54) **IMMUNOGENIC GLYCOPEPTIDES FOR DIAGNOSING PATHOGENIC MICROORGANISMS INFECTIONS**
IMMUNOGENE GLYKOPEPTIDE ZUR DIAGNOSE VON INFEKTIONEN PATHOGENER MIKROORGANISMEN
GLYCOPETIDES IMMUNOGENES POUR LE DIAGNOSTIC D'INFECTIONS PAR DES MICRO-ORGANISMES PATHOGENES

(30) Priority: 30.09.2004 US 953095
(43) Date of publication of application: 25.07.2007
(73) Proprietor: INSTITUT PASTEUR, 75015 Paris (FR)
(72) Inventor: MARCHAL, Gilles, F-94200 IVRY SUR SEINE (FR); ROMAIN, Felix, F-91640 FONTENAY LES BRIS (FR); PESCHER, Pascale, F-92130 ISSY LES MOULINEAUX (FR); BALEUX, Françoise, F-75005 PARIS (FR); SCOTT-ALGARA, Daniel, F-94500 CHAMPIGNY-SUR-MARNE (FR); MULARD, Laurence, F-94270 LE KREMLIN-BICETRE (FR)
(74) Representative: Leblois-Préhaud, Hélène Marthe Georgette
(86) International application number: PCT/IB2005/003303
(87) International publication number: WO 2006/035317

(56) References cited:
- WO-A-02/50108
- US-A1- 2004 171 523
- DOBOS KAREN M ET AL: "DEFINITION OF THE FULL EXTENT OF GLYCOSYLATION OF THE 45-KILODALTON GLYCOPROTEIN OF MYCOBACTERIUM TUBERCULOSIS" JOURNAL OF BACTERIOLOGY, WASHINGTON, DC, US, vol. 178, no. 9, May 1996 (1996-05), pages 2498-2506, XP002180487 ISSN: 0021-9193
- ROMAIN FELIX ET AL: "DEGLYCOSYLATION OF THE 45/47-KILODALTON ANTIGEN COMPLEX OF MYCOBACTERIUM TUBERCULOSIS DECREASES ITS CAPACITY TO ELICIT IN VIVO OR IN VITRO CELLULAR IMMUNE RESPONSES" INFECTION AND IMMUNITY, AMERICAN SOCIETY FOR MICROBIOLOGY. WASHINGTON, US, vol. 67, no. 11, November 1999 (1999-11), pages 5567-5572, XP002180488 ISSN: 0019-9567

### SEQUENCE LISTING

<110> INSTITUT PASTEUR
   MARCHAL, Gilles
   ROMAIN, Felix
   PEACHER, Pascale
   BALEUX, Françoise
   SCOTT-ALGARA, Daniel
   MULARD, Laurence
<120> IMMUNOGENIC GLYCOPEPTIDES FOR DIAGNOSING PATHOGENIC
   MICROORGANISMS INFECTIONS
<130> 226PCT86bis
<160> 12
<170> PatentIn version 3.3
<210> 1
   <211> 39
   <212> PRT
   <213> ARTIFICIAL SEQUENCE
<220>
<223> SYNTHETIC GLYCOPEPTIDE DERIVED FROM MYCOBACTERIUM TUBERCULOSIS
<220>
   <221> MOD_RES
   <222> (10)..(10)
   <223> Glycosidic bond to a di- or a tri- saccharide
<220>
   <221> MOD_RES
   <222> (18)..(18)
   <223> Glycosidic bond to a di- or a tri- saccharide
<220>
   <221> MOD_RES
   <222> (27)..(27)
   <223> Glycosidic bond to a di- or a tri- saccharide
<220>
   <221> MOD_RES
   <222> (39)..(39)
   <223> amidation
<400> 1
   Asp Pro Glu Pro Ala Pro Pro Val Pro Thr Thr Ala Ala Ser Pro Pro
   1               5                   10                  15
   Ser Thr Ala Ala Ala Pro Pro Ala Pro Ala Thr Pro Val Ala Pro Pro
               20                  25                  30
   Pro Pro Ala Ala Ala Asn Thr
           35
<210> 2
   <211> 26
   <212> PRT
   <213> ARTIFICIAL SEQUENCE
<220>
<223> SYNTHETIC GLYCOPEPTIDE DERIVED FROM MYCOBACTERIUM TUBERCULOSIS
<220>
   <221> MOD_RES
   <222> (1)..(1)
   <223> acetylation
<220>
   <221> MOD_RES
   <222> (17)..(17)
   <223> glycosidic bond to a di-or tri-saccharide
<400> 2
   Pro Ala Pro Ala Pro Ala Pro Ala Gly Glu Val Ala Pro Thr Pro Thr
   1               5                   10                  15
   Thr Pro Thr Pro Gln Arg Thr Leu Pro Ala
               20                  25
<210> 3
   <211> 39
   <212> PRT
   <213> artificial sequence
<220>
<223> synthetic glycopeptide derived from Mycobacterium tuberculosis
<220>
   <221> MOD_RES
   <222> (10)..(10)
   <223> alpha-D-man-(1-2)-alpha-D-man-(1-2)
<220>
   <221> MOD_RES
   <222> (18)..(18)
   <223> alpha-D-man-(1-2)-alpha-D-man-(1-2)
<220>
   <221> MOD_RES
   <222> (27)..(27)
   <223> alpha-D-man-(1-2)
<220>
   <221> MOD_RES
   <222> (39)..(39)
   <223> amidation
<400> 3
   Asp Pro Glu Pro Ala Pro Pro Val Pro Thr Thr Ala Ala Ser Pro Pro
   1               5                   10                  15
   Ser Thr Ala Ala Ala Pro Pro Ala Pro Ala Thr Pro Val Ala Pro Pro
               20                  25                  30
   Pro Pro Ala Ala Ala Asn Thr
           35

## Description

The present invention relates to a glycopeptide derived from the Apa protein of *M. tuberculosis,* which can be used for diagnosing tuberculosis.

Infection with *M. tuberculosis* is one of the most serious infections in human medicine. Specifically, 5 to 10 % of individuals infected with *M. tuberculosis* who have a normal immune response develop a serious disease (tuberculosis); this frequency is even higher in individual who have a deficiency in their immune response (infection with HIV, treatment with immuno-suppressors, etc.).

Among the various techniques currently available for diagnosing tuberculosis, mention may be made of:
- the production of pure cultures of *M. tuberculosis,* which is the most rigorous means for diagnosing tuberculosis with certitude. It is a moderately sensitive technique which enables diagnosis for 2/3 of the cases of pulmonary tuberculosis. The results are available only after a minimum delay of 3-4 weeks, sometimes only after culturing for 2 months. The use of culturing techniques employing labelled precursors makes it possible to shorten these delays, which nevertheless remain considerable. This detection of *M. tuberculosis* by culturing requires a sample containing bacilli, which is sometimes difficult to obtain even for pulmonary tuberculosis, in which approximately 1/3 of cases do not receive biological confirmation. Sometimes, this examination requires a specialized medical intervention (lumbar puncture of the cerebrospinal fluid or lymph node biopsy) for extrapulmonary forms of the disease.
- microbiological techniques based on molecular genetics (PCR) are confronted with the same requirement of obtaining a sample containing bacteria. Moreover, because of the presence, in the sample, of PCR reaction inhibitors, the origin of which is impossible to control, these techniques are sometimes unusable. They have not been validated in common practice.
- at the current time, there is no serodiagnosis which has a sensitivity and a specificity compatible with diagnostic use.
- the reaction to tuberculin shows that an individual is sensitized, has been infected with *M. tuberculosis* or has been immunized with BCG. Tuberculin is, in fact, a mixture of *M. tuberculosis* antigens and is therefore incapable of making a distinction between an infection with *M. tuberculosis* and immunization with BCG, because of the very many cross-reactions between the antigens of the vaccine and *M. tuberculosis.* In addition, this reaction to tuberculin does not make it possible to distinguish a tuberculosis, which is an active disease, from an infection with *M. tuberculosis.*

In order to combat tuberculosis more effectively, it would be judicious to have specific diagnostic tools.

The protein named Apa, MPT-32 or 45/47 kDa antigen complex, is a secreted glycoprotein of *M. tuberculosis,* which is encoded by the *Rv 1860* gene (Laqueyrerie et al. Infect. Immun. 1995, 63: 4003-4010). It has been reported that this *M. tuberculosis* Apa molecule contains 6 to 9 mannose residues linked, via a glycosidic bond of the α-(1,2) type, to 4 threonine residues (T₁₀, T₁₈, T₂₇ and T₂₇₇) in the following way: a dimannose (T₁₀ and T₁₈), a mannose (T₂₇), a mannose, a dimannose or a trimannose (T₂₇₇), (Dobos et al., J. Bacteriol., 1996, 178, 2498-2506).

The inventors have previously shown that this protein, which represents only 2 % of the proteins secreted by the bacilli of the tuberculosis group (*M. tuberculosis, M. bovis and BCG*) in culture, is an immunodominant antigen which is very effectively recognized by specific CD4+ T lymphocytes originating from animals infected with *M. tuberculosis* or immunized with BCG (Romain et al., Inf. Immun., 1999, 67, 5567-5572; Horn et al., J. Biol. Chem., 1999, 274, 32023-32030). In these same studies, the inventors also showed that mannosylation of Apa was essential for the antigenic activity of this protein.

The inventors have previously shown also (International PCT Application WO 02/50108 and US Patent Application 2004/0171523) that the oligomannose residues of the side chains of the Apa protein in the native form, produced by *M. tuberculosis* or by live BCG, play a role in the constitution of glycosylated T epitopes (SEQ ID NO: 1, SEQ ID NO: 2) selectively recognized by animals immunized with the live bacteria. The glycopeptides consisting of these glycosylated T epitopes have an antigenic activity at least equal to, if not greater than that of the deglycosylated native protein, the recombinant protein produced in *E. coli* or the conventional antigens (culture of said attenuated live microorganisms, mixtures of antigens prepared from said cultures or non-glycosylated peptides) since they are recognized by a greater number of T lymphocytes specific for live bacilli of the tuberculosis group (*M. tuberculosis* or BCG). Specifically, after immunization with live bacilli of the tuberculosis group, there are many more T lymphocytes specific for the glycopeptides consisting of these glycosylated T epitopes than T lymphocytes specific for the non-glycosylated peptides with the same sequences.

The inventors have now found that another Apa 1-39 glycopeptide (SEQ ID NO: 3) wherein the threonine residues in positions 10, 18 and 27 are bonded to a dimmannose, a dimmannose, a mannose, respectively (Thr₁₀,₁₈(α-D-man-(1-2)-α-D-man-(1-2)), Thr₂₇((α-D-man-(1-2)) is able to differentiate BCG vaccinated healthy individuals from active tuberculosis patients in a T CD4+ lymphocyte assay and is thus useful for diagnosing active tuberculosis or primo-infections with tuberculosis.

A subject of the present invention is a glycopeptide characterized in that it consists of the sequence SEQ ID NO: 3.

Another subject of the present invention is a method *in vitro* for diagnosing tuberculosis, characterized in that it comprises the steps of :
- bringing a biological sample from a patient likely to be infected with *Mycobacterium tuberculosis* into contact with a glycopeptide consisting of SEQ ID NO: 3, derived from the Apa protein of *M. tuberculosis,* and
- detecting CD4+ T lymphocytes recognizing said glycopeptide.

The CD4+ T lymphocytes detection is carried out by any conventional immunology technique which measures the T cell response to an antigen, this technique being known to those skilled in the art. Advantageously, said detection is performed by using a lymphocyte proliferation assay or a cytokine assay (protein or mRNA).

The proliferation assay may be based on Cell-Specific-Fluorescence-Extinction (CSFE) or tritiated thymidine incorporation. The cytokine assay may be carried out by ELISPOT, intracellular cytokine staining, ELISA or RT-PCR. The cytokine which are assayed include : IFN-γ, IL-2, IL-4, IL-5, IL-10, IL-12, IL-15.

The biological sample is a cell suspension containing T CD4+ positive cells. Advantageously, it is a suspension of peripheral blood mononuclear cells (PBMCs). Said biological sample, may be depleted of CD8+ positive cells or pre-enriched in T lymphocytes via a preliminary step of *in vitro* culturing of the cells in the presence of the glycopeptide according to the invention.

According to a preferred embodiment of said method, a biological sample from said patient likely to be infected with *M. tuberculosis,* preferably a PBMCs suspension, is brought into contact with said glycopeptide, and the CD4+ T lymphocytes recognizing said glycopeptide are detected by a lymphocyte proliferation assay or a cytokine assay.

Another subject of the present invention is a kit for diagnosing tuberculosis, characterized in that it comprises a glycopeptide consisting of SEQ ID NO: 3, as defined above.

The following description refers to examples of implementation of the present invention and also to the attached diagrams in which:

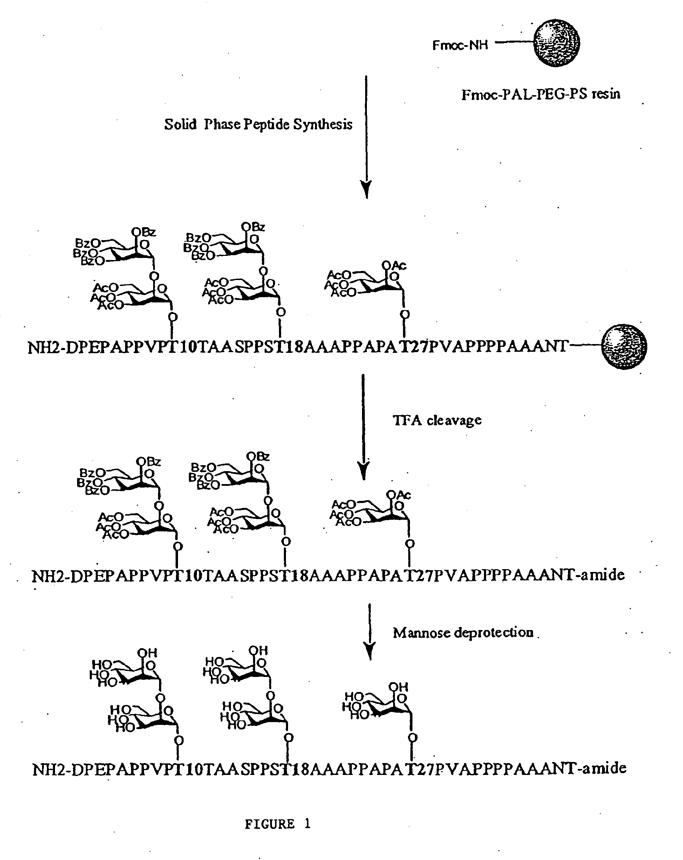

- Figure 1 represents the Thr₁₀,₁₈((α-D-man-(1-2)-α-D-man-(1-2)), Thr₂₇ (α-D-man-(1-2) 1-39 Apa glycopeptide synthesis.

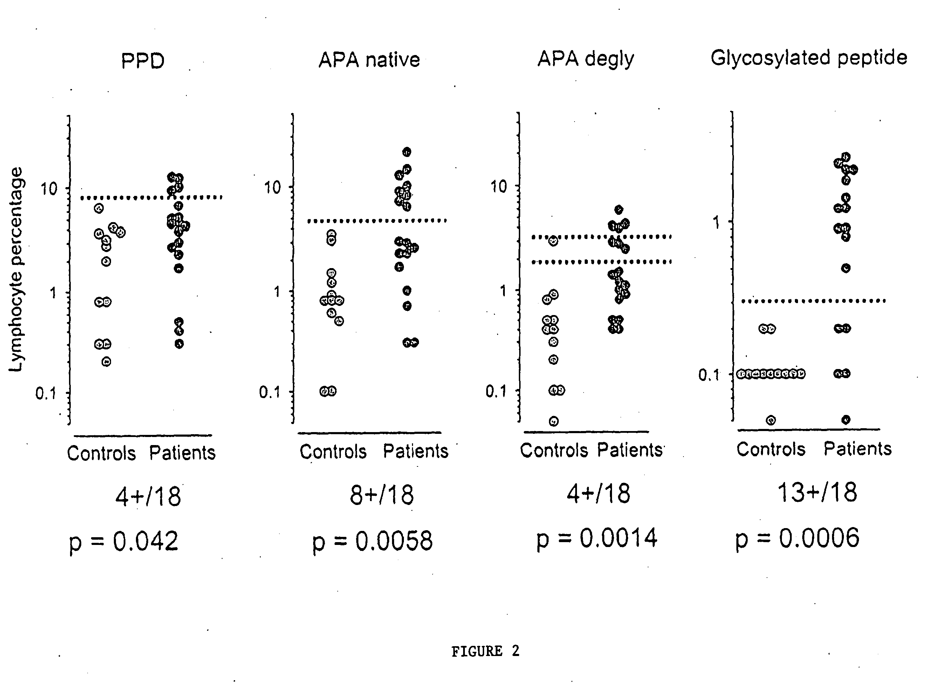

- Figure 2 illustrates CD4+ lymphocytes proliferation of vaccinated subjects (controls) or tuberculosis patients (patients), in the presence of the Apa derived glycopeptide SEQ ID NO: 3, by comparison with native or deglycosylated Apa, and standard purified proteins from *M. tuberulosis* (PPD), measured by cellular specific fluorescence extinction (CSFE). The statistical analysis is performed with the Mann-Whitney test (p value < 0.05 corresponds to a significative difference between the patients and the controls). The proportion of patients which are considered positive in the assay is indicated.

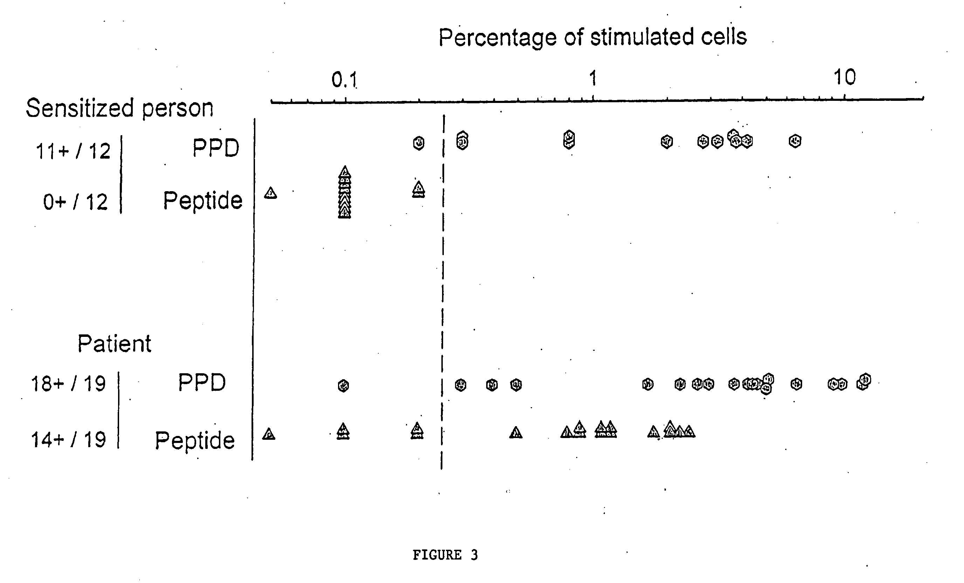

- Figure 3 illustrates CD4+ lymphocytes proliferation of vaccinated subjects (controls) or tuberculosis patients (patients), in the presence of the Apa derived glycopeptide SEQ ID NO: 3, by comparison with standard purified proteins from *M. tuberulosis* (PPD), measured by cellular specific fluorescence extinction (CSFE). The statistical analysis is performed with the Mann-Whitney test (p value < 0.05 corresponds to a significative difference between the patients and the controls). The proportion of patients which are considered positive in the assay is indicated.

### Example 1 : Thr_{10,18} (α-D-man-(1-2)-α-D-man-(1-2)), Thr₂₇(α-D-man-(1-2) 1-39 Apa synthesis.

The 1-39 Apa glycopeptide (SEQ ID NO: 3) was synthesized by solid phase method (Fmoc Solid Phase Peptide Synthesis, a practical approach, W.C. Chan and P.D. White, 2000, Oxford University press) on a fully automated peptide synthesizer (Pioneer®, APPLIED BIOSYSTEMS), using fluorenylmethyloxycarbonyl (Fmoc) chemistry. Starting from 0.1 mmole of Fmoc-PAL-PEG-PS resin (APPLIED BIOSYSTEMS), stepwise elongation of the peptide chain was done twice, using HATU/DIEA activated Fmoc amino acids (4 equivalents). Dimannosylated Threonine_{10,18} (compound 2, figure 1) and monomannosylated Threonine₂₇ (compound 1, Figure 1) were incorporated once, using Dhbt-OH as auxiliary nucleophile (4 equivalents; Jansson et al., J. Chem. Soc., Perkin Trans I, 1992, 1699-1707). These couplings were done manually and completion of the reaction was monitored by the Kaiser ninhydrin test (Fmoc Solid Phase Peptide Synthesis, a practical approach, W.C. Chan and P.D. White, 2000, Oxford University press. Assembling peptide chain yields: 81% (1.01 g of peptide resin). The peptide-resin was then submitted to TFA/TIS/H₂O 95/2.5/2.5 treatment (10 ml/g of resin, 2 hours, room temperature) to give the crude glycopeptide (326 mg, yield: 72 %). Medium Pressure Liquid Chromatogarphy (MPLC) purification on a Nucleoprep 20 µm C18 100 Å preparative column using a 50-100 % linear gradient of acetonitrile in 0.08 % aqueous TFA over 50 minutes at a 25 ml/min flow rate afforded the pure glycopeptide still bearing the protective group on the mannose residues (160 mg, purification yield: 49 %). Deprotection of acetyl and benzoyl groups of the mannose was achieved by sodium methoxide according to known protocol (Jansson et al., J. Chem. Soc., Perkin Trans I, 1992, 1699-17072). Final purification was realized on a 5 µm C18 300 Å semi-preparative column using a 10-25 % linear gradient of acetonitrile in 0.08 % aqueous TFA over 20 minutes at a 6 ml/min flow rate.

The pure Thr_{10,18}(α-D-man-(1-2)-α-D-man-(1-2)), Thr₂₇(α-D-man-(1-2) 1-39 Apa peptide was finally obtained in a 33 % yield (40 mg), leading to a 9 % global yield.

The mass was estimated to 4373.25 ± 0.43 g.mol-1 (expected: 4373.73), by ion spray spectrometry.

A retention time of 15.65 was measured by analytical HPLC (5 µm C18 300 Å Nucleosil analytical column 4,6 X 150 mmm, 10-25 % linear gradient of acetonitrile in 0.08 % aqueous TFA over 20 minutes at a 1 ml/min flow rate).

### EXAMPLE 2: Diagnosis of tuberculosis by a CD4+ T lymphocyte proliferation assay using Thr_{10,18}(α-D-man-(1-2)-α-D-man-(1-2)), Thr₂₇((α-D-man-(1-2) 1-39 Apa glycopeptide.

### 1) Material and Methods

### a) Material

All the monoclonal antibodies are from BECKMAN COULTER: CD45/CD4/CD8/CD3 (# 6607013); CD8-PC5 (# 6607011); CD4-ECD (# 6604727); CD25-RD1 (# 6604422); CD45RO-PE (# IM1307); CD45RA-ECD (# IM2711); TCR PAN-PC5 αβ (# IM2661); TCR PAN-PC5 γδ (#I M2662); CD3-PE (IM 1282); Flow Count (# 7547053); Immunoprep (# 7546999).

### b) Methods

### - PBMC preparation

Peripheral blood mononuclear cells were isolated by Ficoll gradient centrifugation, according to standard protocols. Mononuclear cells numeration, and T lymphocytes, T CD4 + and T CD8+ percentages determination, were determined by flow cytometry, using a panel of antibody, directed to the following cell surface antigens: CD45, CD4, CD8 and CD3.

### - Cell stimulation

The isolated mononuclear cells were cultured The isolated mononuclear cells were cultured for six days in RPMI (GIBCO) supplemented with 20 % AB serum (VALBIOTECH), at 37°C, in a 9 % CO₂ incubator, in the presence or in the absence of one of the following antigen preparations:
- PPD: standard purified proteins from M. tubeculosis (10 mg/ml)
- native Apa (10 µg/ml)
- deglycosylated Apa (10 µg/ml)
- synthetic glycosylated peptide SEQ ID NO: 3 (2 µg/ml). The glycopeptide SEQ ID NO: 3 is one of the glycopeptides defined by SEQ ID NO: 1, wherein the threonine residues in positions 10, 18 and 27 of SEQ ID NO: 1 are bonded to a dimmannose, a dimmannose, a mannose, respectively(Thr_{10,18}(α-D-man-(1-2)-α-D-man-(1-2)), Thr₂₇(α-D-man-(1-2)).

### Stimulation was made in triplicate.

### - Detection of the Apa-specific CDA+ T lymphocytes response in a proliferation assay based on CSFE dilution

Cells previously labelled with .CSFE, according to the manufacturer instructions (BIOPROBES) and stimulated or not, with the different antigen preparations, were incubated for 30 min at room temperature, with the following combinations of antibodies: CD3-PE/CD4-ECD/CD8-PC5; CD45-RA-ECD/CD45-RO-PE/TCRαβb-PC5; CD25-PE/TCRδ-PC5 and fixed with PBS-2 % paraformaldehyde. CD4+ T lymphocytes proliferation was evaluated by measuring the loss of the CFSE fluorescence by flow cytometry.

### - Detection of the Apa-specific CD4+ T lymphocytes response in a proliferation assay based on tritiated thymidine incorporation.

Cells stimulated or not, with the different antigen preparations, were incubated for 18 h in the presence of 1 µCi ³H-thymidine, in RPMI + 10 % AB serum. The cells were then lysed and ³H-thymidine incorporation in the cells was measured by beta-scintillation counting.

### - Detection of the Apa-specific CDA T lymphocyte response in a cytokine-ELISA assay

Two days after the stimulation with the different antigen preparations, the culture supernatant was harvested and the cytokines (IFN-γ, IL-2, IL-4, IL-5, IL-10, IL-12 and IL-15) were assayed by ELISA using commercial kits.

### - Population / Statistical analysis

Twelve BCG-vaccinated healthy individuals (controls) and 18 tuberculosis patients (*M. tuberculosis* infected individuals presenting the clinical signs of tuberculosis disease) were tested. The results were analysed with the Mann-Withney U test, a more sensitive nonparametric alternative to the t-test for independent samples. A p value < 0.05 corresponds to a significant difference between the controls and the patients groups.

### 2) Results

The results of the CD4+ T lymphocytes proliferation assay based on CSFE dilution are presented in figures 2 and 3.

Stimulation with PPD does not allow to differentiate the response from the vaccinated and the tuberculosis patients.

The native Apa antigen stimulates more frequently the response from the patients as compared with the vaccinate controls (8 positive/18 patients); this difference between the groups is lost with the deglycosylated antigen.

By contrast, the glycosylated Apa peptide stimulates the CD4+ T lymphocytes from the majority of patients (13/18 (figure 2); 14/19 (figure 3)) and does not stimulate the CD4+ lymphocytes from the controls.

Therefore, the glycopeptide is useful for diagnosing active tuberculosis or primo-infections with *M. tuberculosis,* in a T CD4+ proliferation assay or in a cytokine production assay.

## Claims

1. A method *in vitro* for diagnosing tuberculosis, **characterized in that** it comprises the steps of:
- bringing a biological sample from a patient likely to be infected with *Mycobacterium tuberculosis* into contact with a glycopeptide consisting of SEQ ID NO: 3, derived from the Apa protein of *M. tuberculosis,* and
- detecting CD4+ T lymphocytes recognizing said glycopeptide.

2. The method according to claim 1, wherein said detection is carried out by a lymphocyte proliferation assay.

3. The method according to claim 1, wherein said detection is carried out by a cytokine assay.

4. The method according to anyone of claims 1 to 3, wherein said biological sample consists of peripheral blood mononuclear cells.

5. A kit for diagnosing tuberculosis,**characterized in that** it comprises at least a glycopeptide consisisting of SEQ ID NO: 3, derived from *M. tuberculosis,* as defined in claim 1.

6. A glycopeptide **characterized in that** it consists of the sequence SEQ ID NO: 3.

## Patentansprüche

1. In-vitro-Verfahren zur Diagnose von Tuberkulose, **dadurch gekennzeichnet, dass** es die Schritte umfasst:
- Inkontaktbringen einer biologischen Probe von einem Patienten, der wahrscheinlich mit *Mycobacterium tuberculosis* infiziert ist, mit einem Glycopeptid, das aus SEQ ID NO: 3 besteht, abgeleitet von dem Apa-Protein von *M. tuberculosis,* und
- Detektieren von CD4+ -T-Lymphozyten, die das Glycopeptid erkennen.

2. Verfahren nach Anspruch 1, wobei die Detektion mittels eines Lymphozytenproliferationsassays durchgeführt wird.

3. Verfahren nach Anspruch 1, wobei die Detektion mittels eines Cytokinassays durchgeführt wird.

4. Verfahren nach einem der Ansprüche 1 bis 3, wobei die biologische Probe aus mononukleären Zellen des peripheren Blutes besteht.

5. Kit zur Diagnose von Tuberkulose, **dadurch gekennzeichnet, dass** er wenigstens ein Glycopeptid umfasst, das aus SEQ ID NO: 3 besteht, abgeleitet von *M. tuberculosis,* wie in Anspruch 1 definiert.

6. Glycopeptid, **dadurch gekennzeichnet, dass** es aus der Sequenz SEQ ID NO: 3 besteht.

## Revendications

1. Méthode *in vitro* pour diagnostiquer la tuberculose, **caractérisée en ce qu'**elle comprend les étapes suivantes:
- mettre en contact un échantillon d'un patient susceptible d'être infecté par *Mycobacterium tuberculosis* avec un glycopeptide consistant en SEQ ID NO : 3, dérivé de la protéine Apa du *M. tuberculosis,* et
- détecter les lymphocytes T CD4+ reconnaissant ledit glycopeptide.

2. Méthode selon la revendication 1, dans laquelle ladite détection est réalisée par un test de prolifération de lymphocytes.

3. Méthode selon la revendication 1, dans laquelle ladite détection est réalisée par une analyse de cytokines.

4. Méthode selon une quelconque des revendications 1 à 3, dans laquelle ledit échantillon biologique est constitué de cellules mononucléaires du sang périphérique.

5. Trousse pour diagnostiquer la tuberculose, **caractérisée en ce qu'**elle comprend au moins un glycopepetide consistant en SEQ ID NO : 3, dérivé du *M. tuberculosis,* tel que défini dans la revendication 1.

6. Glycopeptide **caractérisé en ce qu'**il consiste en la séquence SEQ ID NO : 3.
